# EUROPÄISCHE PATENTSCHRIFT

(11) **EP 0 881 923 B1**
(45) Veröffentlichungstag und Bekanntmachung des Hinweises auf die Patenterteilung: **02.05.2001**
(21) Anmeldenummer: 97901070.9
(22) Anmeldetag: 20.01.1997
(51) Int. Cl.: A62D 3/00, C12R 1/00, B09C 1/00

(54) **BAKTERIENSTAMM COMAMONAS ACIDOVORANS P4a UND VERFAHREN ZUR MIKROBIELLEN DEKONTAMINATION VON MIT PHENOXYESSIGSÄURE-HERBIZIDEN BELASTETEN MATERIALIEN**
BACTERIUM SPECIES COMAMONAS ACIDOVORANS P4a AND PROCESS FOR THE MICROBIAL DECONTAMINATION OF MATERIALS POLLUTED WITH PHENOXYACETIC ACID HERBICIDES
SOUCHE BACTERIENNE COMAMONAS ACIDOVORANS P4a ET PROCEDE DE DECONTAMINATION MICROBIENNE DE MATERIAUX POLLUES PAR DES HERBICIDES A BASE D'ACIDE PHENOXYACETIQUE

(30) Priorität: 22.02.1996 DE 19608319
(43) Veröffentlichungstag der Anmeldung: 09.12.1998
(73) Patentinhaber: UFZ-UMWELTFORSCHUNGSZENTRUM LEIPZIG-HALLE GMBH, 04318 Leipzig (DE)
(72) Erfinder: MÜLLER, Roland, D-04347 Leipzig (DE); BABEL, Wolfgang, D-04347 Leipzig (DE); HOFFMANN, Doreen, D-04860 Torgau (DE)
(74) Vertreter: Ziebig, Marlene, Dr. Dipl.-Chem.
(86) Internationale Anmeldenummer: EP9700241
(87) Internationale Veröffentlichungsnummer: WO9730758

(56) Entgegenhaltungen:
- EP-A- 0 567 102
- DE-C- 4 424 756
- US-A- 4 713 340
- 94TH GENERAL MEETING OF THE AMERICAN SOCIETY FOR MICROBIOLOGY, Bd. 94, Nr. 0, 1994, WASHINGTON, US, Seite 234 XP000197330 C. NAKATSU ET AL. : "Genomic rearrangements observed during experimental evolution of a 2,4-dichlorophenoxyacetic acid degrading Comamonas acidovorans strain TFD41"
- JOURNAL OF CHEMICAL TECHNOLOGY AND BIOTECHNOLOGY, Bd. 61, Nr. 4, - 1994 CHICHESTER, GB, Seiten 299-305, XP000481463 K-H OH AND O.H. TOUVINEN: "Microbiological treatment of fertilizer solid waste material containing phenoxyalkanoic herbicides 2,4-D and MCPP"
- ACTA BIOTECHNOLOGICA, Bd. 16, Nr. 2-3, 21.August 1996, BERLIN, DE, Seiten 121-131, XP000197329 D. HOFFMANN ET AL.: "Isolation and characterization of an alkaliphilic bacterium capable of growing on 2,4-dichlorophenoxyacetic acid and 4-chloro-2-methylphenoxyacetic acid"

## Beschreibung

Die Erfindung betrifft den neuen alkaliphilen Bakterienstamm *Comamonas acidovorans* P4a und ein Verfahren zur mikrobiellen Dekontamination von Herbizid-belasteten Materialien und Wässern unter neutralen bis stark alkalischen Bedingungen, die mit 2,4-Dichlor- und/oder 4-Chlor-2-methylphenoxyessigsäure oder deren Salzen kontaminiert sind. Das Verfahren kann zur Sanierung von Gebäudeabbruch, beispielsweise von Altstandorten der chemischen Industrie bzw. Gebäuden zur Lagerung und zum Umschlag solcher Substanzen, oder zur Reinigung alkalischer Wässer aus der Produktion solcher Herbizide oder zur Reinigung von mit diesen Herbiziden verunreinigten Böden, Gewässern und Grundwässern Anwendung finden.

Mauerwerk von Gebäuden, in denen jahrzehntelang Herbizide produziert worden sind, ist hochgradig kontaminiert. Die Kontamination umfaßt das Spektrum der respektiven Ausgangs-, Zwischen- und Endprodukte. Wäßrige Eluate aus solchen Materialien sind stark alkalisch. Stark alkalische wäßrige Abproduktströme, die mit diesen Substanzen belastet sind, fallen auch bei der Produktion der Phenoxyalkansäuren und deren Salzen an. Die Belastung erstreckt sich auch auf das Umfeld solcher (ehemaligen) Standorte. Die Kontamination findet man in Böden und Grundwässern, der pH-Wert bewegt sich häufig ebenfalls im alkalischen Bereich. Diese Verbindungen sind toxisch und haben z.T. cancerogene und teratogene Wirkung. Ziel ist ihre Beseitigung, was zur Vermeidung gesundheitlicher Schäden von exponierten Personen und zum Schutz der Umwelt unbedingt erforderlich ist.

Nach dem Abbruch solcher Gebäude und Anlagen und der Zerkleinerung des Mauerwerks über Schredderanlagen fallen entsprechend kontaminierte Materialien an. Vor einer weiteren Verwendung solchen Materials müssen praktikable und kostengünstige Maßnahmen zur Dekontamination vorgenommen werden. Das betrifft gleichermaßen die Entsorgung von Wässern aus der Produktion solcher Verbindungen, die diese Komponenten in konzentrierter Form beinhalten. Es hat sich gezeigt, daß die Einleitung solcher Produktionswässer in industrielle Kläranlagen zu signifikanten Störungen des biologischen Gleichgewichts und damit der Leistungsfähigkeit dieser Anlagen mit Auswirkungen auf die Verweilzeit und den Abbaugrad führen kann. Eine effektive Lösung dieses Problems erfordert spezielle Bedingungen und ist an Voraussetzungen geknüpft.

Zur Dekontamination stehen verschiedene Verfahren auf physikalisch-chemischer Grundlage zur Verfügung. Thermische Verfahren sind zwar effektiv, aber kostenintensiv. Absaug- oder Waschverfahren mit anschließender adsorptiver Schadstoffentfernung, auch im Falle belasteter Wässer, sind eine andere Möglichkeit, verlagern das Problem aber nur aufeinen anderen Träger, wenn auch in z.T. hoch konzentrierter Form.

In mikrobiellen Verfahren wird prinzipiell eine effektive und kostengünstigere Variante gesehen. Hierbei werden die organischen Verbindungen durch die Aktivitäten der Mikroorganismen unter Bildung von Biomasse, Wasser, Kohlendioxid und Wärme abgebaut. Dazu können die am Standort angesiedelten und an das entsprechende Milieu adaptierten Mikroorganismen genutzt werden.

Es können aber auch ex situ kultivierte Mikroorganismen (Spezies oder Konsortien) als Starterkulturen kontaminierten Materialien zugesetzt werden, wobei die Sanierungen in unterschiedlichsten Verfahrensregimen durchgeführt werden können (d.h. in situ, on site, off site, Reaktoren, Mieten u.d.g.). Für belastete und konzentrierte Wässer bietet sich eine separierte Behandlung und Degradation in Reaktoren an.

Die produktive Dekontamination von chlorierten und methylierten Phenolen und Phenoxyalkansäuren im neutralen pH-Bereich durch Einzelkulturen (Pieper, D.H. et al. 1988, Arch. Microbiol. 150, 95; Horvath, M. et al. 1990, Appl. Microbiol. Biotechnol. 33, 213; Kilpi, S. 1980, Microbiol. Ecol. 6, 261; Short, K.A. et al. 1990, Can. J. Microbiol. 36, 822; Tiedje, J.M. et al. 1969, J. Agr. Food Chem. 17, 1021) und Konsortien (Bloedorn, I. 1990, Dissertation, Universität Halle; Haugland, R.A. et al. 1990, Appl. Env. Microbiol. 56, 1357; Lappin, H.M. 1985, Appl. Env. Microbiol. 49, 429; Oh, K.H. und Tuovinen, O.H. 1990, J. Ind. Microbiol. 6, 275;) ist bekannt. Für das betrachtete Umfeld kommt neben o.g. Faktoren als erschwerender Umstand hinzu, daß Eluate aus solchen Materialien auf Grund des alkalischen Moduls dieser Stoffe pH-Werte von bis zu 12,5 aufweisen. Dies sind Bedingungen, die die Artenvielfalt stark einschränken. Es ist das Milieu der sog. Alkaliphilen (K. Horikoshi "Microorganisms in Alkaline Environments", VCH Weinheim, New York 1991).

Jüngst konnte gezeigt werden, daß Mikroorganismen, die aus solch kontaminiertem Mauerwerk angereichert worden waren, als Konsortien zum vollständigen Abbau unterschiedlicher Phenoxyalkansäure-Derivate in wäßrigen Eluaten mit pH-Werten bis zu 12,5 befähigt sind (Müller et al., DE-PS-4424756.7).

Es konnten aber bislang keine Organismen nachgewiesen werden, die in Reinkultur in der Lage sind, im stark alkalischen Milieu solche chlorierten und methylierten Phenoxyalkansäuren, insbesondere 2,4-D und MCPA, als Kohlenstoff- und Energiequelle zu nutzen und damit produktiv abzubauen. Solche axenische Kulturen sind im Vergleich zu Konsortien in ihrem metabolischen Spektrum zwar eingeschränkt, was dann aber kein Nachteil ist, wenn sie über eine der Kontamination adäquate Stoffwechselleistung verfügen. Dagegen haben sie aber den großen Vorteil der definierteren Handhabbarkeit.

Aufgabe der Erfindung war es, einen Mikroorganismenstamm zur Verfügung zu stellen, der in Reinkultur zur Dekontamination von mit Zwischen- und Endprodukten der Herbizidproduktion, insbesondere mit 2,4-Dichlorphenoxyessigsäure (2,4-D) und 4-Chlor-2-methylphenoxyessigsäure (MCPA) bzw. mit deren Salzen kontaminierten Materialien, wie Abbruchmaterialien von Gebäuden und Anlagen, Böden, Produktionsabwässern oder Grundwässern eingesetzt werden kann und der es erlaubt, ein produktives Dekontaminationsverfahren für die genannten Herbizide zu etablieren.

Diese Aufgabe wird erfindungsgemäß durch den als *Comamonas acidovorans* identifizierten Stamm P4a gelöst. Dieser Stamm wächst im leicht sauren bis alkalischen Bereich auf 2,4-D und MCPA bzw. deren Salzen als einziger Kohlenstoff- und Energiequelle und ist bis zu pH-Werten von 12,5 stabil und stoffwechselaktiv. Somit ist es möglich, die oben bezeichneten Herbizide auch in sehr stark alkalischem Bereich abzubauen und z.B. das betroffene Mauerwerk oder entsprechende wäßrige Medien damit zu dekontaminieren.

Der Stamm wurde aus Herbizid-belastetem Mauerwerk isoliert und am 9.01.1996 bei der Deutschen Sammlung von Mikroorganismen und Zellkulturen GmbH unter der Nummer DSM 10474 hinterlegt.

Der Stamm wird ex situ bei pH-Werten von 6 bis 11, vorzugsweise 8 bis 10, auf 2,4-D und/oder (MCPA) oder deren Salzen vermehrt, wobei die Vermehrung gegebenenfalls durch Zugabe einer komplexen Kohlenstoff- und Energiequelle stabilisiert werden kann.

Das erfindungsgemäße Dekontaminationsverfahren unter Verwendung des Stammes P4a bzw. die Vermehrung des Stammes P4a kann zum einen kontinuierlich erfolgen, indem man wäßrige Eluate von mit 2,4-D und/oder MCPA belasteten Materialien oder 2,4-D und/oder MCPA belastete Wässer in einer Verdünnungsrate von bis zu 0,2 h⁻¹ einer bei alkalischen pH-Werten, vorzugsweise pH 8,5 bis 9,5, wachsenden Kultur von *Comamonas acidovorans* P4a in einer Konzentration von rund 10 Gewichtsteilen pro Gewichtsteil Bakterientrockenmasse zuführt. Essentielle Wachstumskomponenten wie Stickstoff und Phosphor müssen in adäquater Konzentration vorhanden sein. Unter diesen Bedingungen erfolgt eine quantitative Degradation dieser Herbizide unter Wandlung in bakterielle Biomasse, Wasser, CO₂, HCl und Wärme. Das Wachstum kann durch Zusatz von bis zu 0,25 Gewichtsteilen pro Gewichtsteil Herbizid einer komplexen Kohlenstoffquelle wie z.B. Hefeextrakt unterstützt werden, der Zusatz weiterer essentieller Wachstumselemente kann hierbei entfallen.

Das erfindungsgemäße Dekontaminationsverfahren bzw. die Vermehrung des Stammes P4a kann auch diskontinuierlich erfolgen, wobei in die Erzeugung der Biomasse ein Wachstumsschritt unter Nutzung eines an sich üblichen Nährmediums mit einer komplexen Kohlenstoff- und Energiequelle mit einer anschließenden Konditionierung des Stammes eingeschlossen sein kann. Die Konditionierung kann vorteilhafterweise nach Vermehrung des Stammes auf einem 2,4-D und/oder MCPA enthaltenden Medium oder Material (d.h. in situ) durchgeführt werden.

Die diskontinuierliche Dekontamination erfolgt also beispielsweise so, daß man eine extern auf 2,4-D und/oder MCPA - vorzugsweise in Gegenwart von bis zu 0,25 Gewichtsanteilen Hefeextrakt pro Gewichtsanteil Herbizid - bei pH 8 bis 10 kultivierte Bakterienbiomasse zusetzt. Die Vermehrung der Biomasse kann auch so erfolgen, daß man eine auf 2,4-D und/oder MCPA kultivierte und anschließend auf einem komplexen Wachstumssubstrat - vorzugsweise Hefeextrakt, Pepton und Fructose in einem Verhältnis von 1:1:0,5 - über 24 bis 48 h weiter vermehrte und anschließend bis zu 24 h auf 2,4-D und/oder MCPA - vorzugsweise 0,2 g/l - weiter behandelte Biomasse einsetzt. Die Biomasse soll hierbei in einer Konzentration von 0,1 bis 1 g/l oder kg einem mit 2,4-D und/oder MCPA in einer Konzentration bis zu 2 g/l kontaminiertem Material in wäßrigem Milieu, welches pH-Werte bis zu 12,5 aufweisen kann, zugesetzt werden.

Unter solchen Bedingungen erfolgt die Dekontamination von bis zu 0,4 g/l 2,4-D bei einer Biomassekonzentration von 0,7 g/l bei pH-Werten bis zu 9 innerhalb von 24 h, bei pH-Werten bis zu 11 innerhalb von 3 Tagen und bei pH-Werten bis zu 12 innerhalb von 5 Tagen.

Bei einer 2,4-D Konzentration von 0,8 g/l erfolgt eine komplette Degradation innerhalb eines Tages bei pH-Werten bis zu 9, innerhalb von 2 Tagen bei pH-Werten bis zu 10 und innerhalb von 5 Tagen bei pH-Werten bis zu 11. Selbst bei einer 2,4-D Konzentration von 1,6 g/l wird kompletter Abbau bei pH-Werten von 11 noch innerhalb von 7 Tagen erreicht.

Die Abbauleistungen unter diesen batch-Bedingungen sind insofern überraschend, als Wachstum nur bis zu pH-Werten von 11 beobachtet wird und die stationären Substratkonzentrationen auf Grund der gewählten Kultivierungsbedingungen in weiten Bereichen < 0,1 g/l sind. Der Stamm ist offensichtlich in der Lage, durch Konditionierung des Milieus, d.h. Absenkung des externen pH-Wertes auf Werte zwischen 9 und 10, die Möglichkeiten seines Überlebens über Stoffwechslaktivitäten aktiv zu beeinflussen und durch Wachstum und Vermehrung entsprechend höhere externe Konzentrationen dieser toxischen Verbindungen zu tolerieren.

Es war insgesamt überraschend, daß so eine hohe pH-Resistenz erreicht wird und daß solch eine hohe Belastung an diesen toxischen Verbindungen toleriert wird. Das ist so für neutrophile Mikroorganismen im Zusammenhang mit dem Abbau derartiger Herbizide bisher nicht beobachtet worden.

Die Degradation der Herbizide durch Wachstum und Vermehrung erfolgt unter aeroben Bedingungen.

Es ist vorteilhaft, ein Temperaturregime von 4 bis zu 38°C, vorzugsweise von 20 bis 30°C, zu wählen.

Das Dekontaminierungsverfahren kann auch so gestaltet werden, daß Zellen von *Comamonas acidovorans* P4a auf oberflächenvergrößernde Strukturen, z.B. auf oberflächenbehandelte Polyurethan-Pellets, aufgetragen werden. Auf diesen kommt es zu einem Bewuchs, der vor allem nach Beimpfung mit ex situ erzeugter bakterieller Biomasse nach einmaligem batch-Betrieb zu vergleichbaren Abbauraten führt. Mit 2,4-D und/oder MCPA belastete wäßrige Medien können über solch einen Festbettreaktor geleitet werden und der Abbau kontinuierlich gestaltet werden. Die Zulaufraten, die einen vollständigen Abbau ermöglichen, werden vom Belastungsgrad und der Kapazität des Festbettreaktors bestimmt. Bei Langzeitbetrieb, auch unter dem Aspekt einer industriellen Dimension, kann durch entsprechend weitergehenden Bewuchs bzw. Erhaltung eines hohen Aktivitätspotential dieses Stammes die Zeit für den Abbau der Kontaminanten sogar weiter verringert werden.

Durch nachfolgende Ausführungsbeispiele soll das erfindungsgemäße Vefahren näher erläutert werden, ohne es jedoch darauf einzuschränken:

### Beispiel 1 - Kultivierung des erfindungsgemäßen Stammes

Der Stamm *Comamonas acidovorans* P4a wird auf einem Minimalmedium angezogen. Dieses Medium hat pro Gramm zu erzeugender Biomasse folgende Zusammensetzung (in mg/l) :

NH₄Cl, 700; KH₂PO₄158; MgSO₄*7 H₂O, 8; CaCl₂* 2 H₂O, 10; FeSO₄* 7 H₂O, 2,5; ZnSO₄* 7 H₂O, 0,23; MnSO₄* 4 H₂O, 0,42; CuSO₄* 5 H₂O, 0,39; Na₂MoO₄, 0, 12, NaCl, 3000. Die Kultivierung erfolgt im pH-Bereich von 8 bis 10, bei 30°C und einer Gelöstsauerstoffkonzentration von > 30% des Luftsättigungswertes. Als Kohlenstoff- und Energiequelle dienen 2,4-D und/oder MCPA bzw. deren Salze. Es kann auch unmittelbar ein wäßriges, die Komponenten 2,4-D und/oder MCPA enthaltendes Medium als Kohlenstoff- und Energiequelle zugefüttert werden.
Hefeextrakt kann zur Unterstützung des Wachstums in einer Konzentration von 0,25 Gewichtsanteilen pro Gewichtsanteil Herbizidsubstrat zusätzlich angeboten werden. Die Wachstumsraten werden auf 0,1 bis 0,2 h⁻¹ eingestellt. Nach Erreichen stabiler Wachstumsbedingungen kann diese Kultur zur Dekontamination von mit Phenoxyessigsäure-Derivaten belasteten Wässern eingesetzt werden.

### Beispiel 2- Kontinuierliches Dekontaminationsverfahren

Mit 2,4-D und/oder MCPA belastete wäßrige Medien werden bei kontinuierlichen, über Wachstum gekoppelten Degradationsverfahren mit einer Verdünnungsrate bis zu 0,2 h⁻¹ zugeführt. Der pH-Wert wird bei stabiler Prozeßführung im Bereich von 7 bis 10, vorzugsweise 8 bis 9, gehalten. Essentielle Wachstumskomponenten, insbesondere Stickstoff, Phosphor, und Natrium, müssen gegebenenfalls und in adäquanten Mengen vorhanden sein. Der Bedarf an Spurenelementen kann bei Zuführung natürlicher Wässer vernachlässigt werden. Komplexe Kohlenstoff- und Energiequellen können zur Stabilisierung des Wachstums zugeführt werden. Unter diesen Voraussetzungen wird eine komplette Degradation von 2,4-D und/oder MCPA bei diesen Geschwindigkeiten erreicht. Der Abbau wird über spektrophotomerische oder chromatographische (HPLC) Methoden bzw. über die Freisetzung von z.B. Chlorid verfolgt.

### Beispiel 3 - Diskontinuierliches Dekontaminationsverfahren

Die Degradation von belasteten Materialien und Wässern kann auch diskontinuierlich gestaltet werden. Comamonas acidovorans P4a wird dazu kontinuierlich nach oben beschriebener Verfahrensweise auf 2,4-D und/oder MCPA als Kohlenstoff- und Energiequelle oder diskontinuierlich durch sequentielle Zugabe von 2,4-D oder MCPA in Konzentrationen von 0,2 bis 0,5 g/l kultiviert. Eine weitere Vermehrung solch einer auf einem der beiden Herbizide oder beiden Herbiziden vorkultivierten Biomasse kann durch Wachstum in einer 24- bis 48-stündigen Passage auf einem komplexen Wachstumsmedium, welches z.B. Hefeextrakt, Pepton und Fructose in einem Gewichtsverhältnis von 1:1:0,5 enthält, vorgenommen werden. An diese Wachstumsphase schließt sich eine Phase der Konditionierung durch Zugabe von 0,2 bis 0,5 g/l 2,4-D oder MCPA an. Nach 24 h wird diese Biomasse oder eine wie oben beschrieben kontinuierlich erzeugte Biomasse zur Dekontamination von 2,4-D und/oder MCPA belasteten wäßrigen Medien eingesetzt. In einer Konzentration von 0,7 g Bakterientrockenmasse/l erfolgt eine Degradation von 2,4-D oder MCPA in einer Konzentration von 0,8 g/l bei pH-Werten bis zu 9 innerhalb von 2 Tagen, bei pH-Werten bis zu 11 innerhalb von 7 Tagen. Bei einer Konzentration der Herbizide von 0,4 g/l erfolgt der Abbau innerhalb von 5 Tagen selbst noch bei pH-Werten von 12. Der Zeitbedarf ist proportional der Biomassekonzentration. Bei einer Konzentration von 0,1 g Bakterienkonzentration/l erhöht sich die Zeit für einen vollständigen Abbau von 0,4 g/l 2,4-D bei einem pH von 10 auf 3 Tage und bei einem pH von 11 auf 6 Tage. Ähnliche Werte für eine komplette Degradation in Abhängigkeit vom pH-Wert werden bei MCPA erhalten.

## Patentansprüche

1. Alkaliphiler Bakterienstamm *Comamonas acidovorans* P4a (DSM 10474).

2. Verfahren zur mikrobiellen Dekontamination von mit 2,4-Dichlorphenoxyessigsäure (2,4-D) und/oder 4-Chlor-2-methylphenoxyessigsäure (MCPA) oder deren Salzen belasteten Materialien oder Wässern,
dadurch gekennzeichnet, daß
zur Dekontamination der alkaliphile Bakterienstamm *Comamonas acidvorans* P4a (DSM 10474) eingesetzt wird und die Dekontamination in Gegenwart von für die Gattung Comamonas üblichen Wachstumskomponenten bei pH-Werten von 6 bis 12,5 und Temperaturen von 4-38°C unter aeroben Bedingungen durchgeführt wird.

3. Verfahren nach Anspruch 2,
dadurch gekennzeichnet, daß
die Dekontamination in wäßrigen Eluaten aus den belasteten Materialien oder von solchen Materialien in befeuchtetem Zustand erfolgt.

4. Verfahren nach Anspruch 2 oder 3,
dadurch gekennzeichnet, daß
der Bakterienstamm *Comamonas acidovorans* P4a ex situ bei pH-Werten von 6 bis 11, vorzugsweise von 8 bis 10, auf 2,4-D und/oder MCPA oder deren Salzen vermehrt wird und die Vermehrung gegebenenfalls durch Zugabe einer komplexen Kohlenstoff- und Energiequelle stabilisiert wird.

5. Verfahren nach Anspruch 4,
dadurch gekennzeichnet, daß
die Vermehrung des Stammes *Comanonas acidovorans* P4a kontinuierlich bei Verdünnungsraten bis zu 0,2 h⁻¹ erfolgt, wobei gleichzeitig die wäßrigen Eluate aus den belasteten Materialien kontinuierlich dekontaminiert werden.

6. Verfahren nach Anspruch 4,
dadurch gekennzeichnet, daß
die Vermehrung des Stammes *Comamonas acidovorans* P4a diskontinuierlich erfolgt und in die Erzeugung der Biomasse ein Wachstumsschritt unter Nutzung eines an sich üblichen Nährmediums mit einer komplexen Kohlenstoff- und Energiequelle mit einer anschließenden Konditionierung des Stammes eingeschlossen ist.

7. Verfahren nach Anspruch 6,
dadurch gekennzeichnet, daß
die Konditionierung nach Vermehrung des Stammes auf einer komplexen Kohlenstoff- und Energiequelle auf einem 2,4-D und/oder MCPA enthaltenden Medium oder Material durchgeführt wird.

8. Verfahren nach einem der Ansprüche 6 oder 7,
dadurch gekennzeichnet, daß
die Dekontamination diskontinuierlich erfolgt, indem Biomasse von *Comamonas acidovorans* P4a belasteten Materialien oder Wässern in einer Konzentration von 0,1 bis 1 g pro Liter oder kg zugesetzt wird.

9. Verfahren nach einem der Ansprüche 2 bis 8,
dadurch gekennzeichnet, daß
die Dekontamination in einem Festbettreaktor durchgeführt wird.

## Claims

1. The alkaliphilic bacterial strain *Comamonas acidovorans* P4a (DSM 10474).

2. A process for the microbial decontamination of materials or waters polluted with 2,4-dichlorophenoxyacetic acid (2,4-D) and/or 4-chloro-2-methylphenoxyacetic acid (MCPA) or salts thereof, characterized in that the alkaliphilic bacterial strain *Comamonas acidovorans* P4a (DSM 10474) is used in said decontamination, said decontamination being carried out in the presence of growth components conventionally used for the *Comamonas* genus and at pH values of from 6 to 12.5 and temperatures of from 4 to 38°C under aerobic conditions.

3. The process according to claim 2, characterized in that the decontamination is performed in aqueous eluates from polluted materials or on such materials in wetted condition.

4. The process according to claim 2 or 3, characterized in that the *Comamonas acidovorans* P4a bacterial strain is grown *ex situ* on 2,4-D and/or MCPA or salts thereof at pH values of from 6 to 11, preferably from 8 to 10, said growth optionally being stabilized by adding a complex carbon and energy source.

5. The process according to claim 4, characterized in that the *Comamonas acidovorans* P4a strain is grown continuously at dilution rates of up to 0.2 h⁻¹, the aqueous eluates from the polluted materials being decontaminated continuously at the same time.

6. The process according to claim 4, characterized in that the *Comamonas acidovorans* P4a strain is grown in a discontinuous fashion, the production of biomass comprising a growth step utilizing a *per se* usual nutrient medium including a complex carbon and energy source, with subsequent conditioning of the strain.

7. The process according to claim 6, characterized in that said conditioning is performed after growing the strain on a complex carbon and energy source on a medium or material containing 2,4-D and/or MCPA.

8. The process according to any of claims 6 or 7, characterized in that decontamination is effected discontinuously by adding biomass of *Comamonas acidovorans* P4a to polluted materials or waters at a concentration of from 0.1 to 1 g per liter or kg.

9. The process according to any of claims 2 to 8, characterized in that the decontamination is performed in a fixed-bed reactor.

## Revendications

1. Souche bactérienne alcaliphile *Comamonas acidovorans* P4a (DSM 10474).

2. Procédé de décontamination microbienne de matières ou d'eaux chargées en 2,4 dichlorophénoxyacétate (2,4 D) et/ou 4-chlore-2-méthylphénoxyacétate (MCPA), ou en sels desdites substances,
**caractérisé en ce que**
il est recouru à la souche bactérienne alcaliphile *Comamonas acidovorans* P4a (DSM 10474) pour la décontamination, et en ce que celle-ci s'effectue sous conditions aérobies en présence des composants de croissance usuelles au genre Comamonas, avec une valeur pH comprise entre 6 et 12,5 et des températures comprises entre 4 et 38 °C.

3. Procédé selon revendication 2,
**caractérisé en ce que**
la décontamination est réalisée dans des éluats aqueux provenant des matières chargées, ou desdites matières à l'état humide.

4. Procédé selon revendication 2 ou 3,
**caractérisé en ce que**
la souche bactérienne *Comamonas acidovorans* P4a est reproduite *ex situ* avec une valeur pH comprise entre 6 et 11, préférentiellement entre 8 et 10, sur 2,4 D et/ou MCPA ou les sels de ces derniers, et en ce que la reproduction est stabilisée le cas échéant par addition d'une source complexe de carbone et d'énergie.

5. Procédé selon revendication 4,
**caractérisé en ce que**
la reproduction de la souche *Comamonas acidovorans* P4a s'effectue de manière continue avec un taux de dilution pouvant atteindre 0,2 h⁻¹, les éluats aqueux provenant des matières chargées étant simultanément décontaminés de manière continue.

6. Procédé selon revendication 4,
**caractérisé en ce que**
la reproduction de la souche *Comamonas acidovorans* P4a s'effectue de manière discontinue et qu'une phase de croissance est comprise dans la production de la biomasse par recours à un milieu nutritif usuel en soi avec une source complexe de carbone et d'énergie, avec conditionnement consécutif de la souche.

7. Procédé selon revendication 6,
**caractérisé en ce que**
le conditionnement consécutif à la reproduction de la souche sur une source complexe de carbone et d'énergie est effectué sur un milieu ou une matière à teneur en 2,4 D et/ou MCPA.

8. Procédé selon l'une des revendications 6 ou 7,
**caractérisé en ce que**
la décontamination s'effectue de manière discontinue, une biomasse de *Comamonas acidovorans* P4a dans une concentration de 0,1 à 1 g par kg ou litre étant additionnée à la matière ou l'eau chargée.

9. Procédé selon l'une des revendications 2 à 8,
**caractérisé en ce que**
la décontamination est effectuée dans un réacteur à lit fixe.
